(19) Europäisches Patentamt

European Patent Office

Office européen des brevets

(11) EP 0 918 080 B1

(12) EUROPÄISCHE PATENTSCHRIFT

(45) Veröffentlichungstag und Bekanntmachung des Hinweises auf die Patenterteilung:
14.05.2003 Patentblatt 2003/20

(51) Int Cl.⁷: **C09J 7/04**, A61L 15/58

(21) Anmeldenummer: 98120787.1

(22) Anmeldetag: 03.11.1998

(54) **Partiell selbstklebend ausgerüstetes Trägermaterial**

Carrier with discontinuous self-adhesive coating

Support avec revêtement auto-adhésif discontinu

(84) Benannte Vertragsstaaten:
AT DE ES FR GB IT NL SE

(30) Priorität: 22.11.1997 DE 19751873

(43) Veröffentlichungstag der Anmeldung:
26.05.1999 Patentblatt 1999/21

(73) Patentinhaber: Beiersdorf Aktiengesellschaft
20245 Hamburg (DE)

(72) Erfinder:
• **Himmelsbach, Peter**
**21614 Buxtehude (DE)**
• **Lehder, Matthias**
**21244 Buchholz (DE)**
• **Jauchen, Peter**
**22455 Hamburg (DE)**
• **Keite-Telgenbüscher, Klaus, Dr.**
**22529 Hamburg (DE)**

(56) Entgegenhaltungen:
EP-A- 0 356 777          DE-A- 4 308 649
DE-A- 19 620 107

• DATABASE WPI Derwent Publications Ltd., London, GB; AN 96-027049 XP002093468 & JP 07 300575 A (NAKAMURA SEISHISHO KK) 14. November 1995

**Beschreibung**

Die Erfindung betrifft ein zumindest einseitig partiell selbstklebend ausgerüstetes Trägermaterial, das mit einer Selbstklebemasse in Form geometrischer Körper beschichtet worden ist, Verfahren zur Herstellung des beschichteten Trägermaterials sowie dessen Verwendung.

Die partielle Beschichtung von Trägermaterialien mit druckempfindlichen Selbstklebemassen ist eine bekannte Technik, sei es, die Selbstklebemassen rasterpunktförmig, beispielsweise durch Siebdruck (DE-PS 42 37 252), wobei die Klebstoffkalotten auch unterschiedlich groß und/oder unterschiedlich verteilt sein können (EP-PS 353 972), oder durch Tiefdruck von in Längs- und Querrichtung zusammenhängenden Stegen aufzubringen (DE-PS 43 08 649). Weiter ist bekannt, daß speziell rückstandsfrei ablösbare Trägermaterialien mit Selbstklebematerialien beschichtet werden können.

In DE-OS 42 37 252 werden spezielle Geometrien zur Erreichung der Rückstandsfreiheit verwendet.

Eine Dotierung von partiell beschichteten Selbstklebebändern ist auch beschrieben. US 4 699 792 beschreibt eine solche Pflastervorrichtung mit aktiven Substanzen.

Im Handel sind rückstandsfrei wiederablösbare Flächengebilde in Blockform u.a. unter dem Namen "tesa Notes" ® von der Firma Beiersdorf erhältlich.

Als Trägermaterialien sind bereits zahlreiche Materialien auf Folien-, Gewebe-, Gewirke-, Vlies-, Gel- oder Schaumstoffbasis vorbeschrieben und werden auch in der Praxis eingesetzt.

Auf dem medikalen Sektor stellen sich besondere Anforderungen an die Trägermaterialien. Die Materialien müssen hautverträglich, in der Regel luft- und wasserdampfdurchlässig sowie gut anmodellierbar und anschmiegsam sein. Aufgrund dieser Anforderungen wird häufig ein möglichst dünner oder weicher Träger bevorzugt. Zur Handhabung und beim Gebrauch ist bei den Trägermaterialien aber auch eine ausreichende Festigkeit und gegebenenfalls begrenzte Dehnbarkeit gefordert. Weiterhin sollte das Trägermaterial auch nach dem Durchnässen eine ausreichende Festigkeit und geringe Dehnbarkeit aufweisen.

Für spezielle Anwendungen, zum Beispiel Tapes für funktionelle Tapeverbände zur Prophylaxe und Therapie von Verletzungen, Erkrankungen und Veränderungen am Bewegungsapparat, sind unelastische Träger mit einer hohen Festigkeit in Beanspruchungsrichtung erforderlich. Dies wird erreicht, indem Gewebe, üblicherweise aus Baumwolle oder Viskose, eingesetzt werden. In der Regel sind solche Trägermaterialien, mit entsprechend hohem Flächengewicht, kostenintensiv. Eine hohe Flexibilität ist nur durch ein Gewebe mit geringerer Festigkeit zu erreichen. Dieses weist im allgemeinen aber dann unter Beanspruchung eine gewisse Dehnung auf, welche für die Anwendung unerwünscht ist.

Der Vorteil des rasterförmigen Auftrags besteht darin, daß die Klebematerialien bei entsprechend porösem Trägermaterial luft- und wasserdampfdurchlässig sowie in der Regel leicht wieder ablösbar sind.

Der partielle Auftrag ermöglicht insbesondere bei medikalen Anwendungen durch geregelte Kanäle die Abführung des transepidermalen Wasserverlustes und verbessert das Ausdampfen der Haut beim Schwitzen insbesondere bei der Verwendung von luft- und wasserdampfdurchlässigen Trägermaterialien. Hierdurch werden Hautirritationen, die durch Stauungen der Körperflüssigkeiten hervorgerufen werden, vermieden. Die angelegten Abführungskanäle ermöglichen ein Ableiten auch unter Verwendung eines mehrlagigen Verbandes.

Ein Nachteil dieser Produkte besteht jedoch darin, daß bei zu hoher Flächendeckung der an sich undurchlässigen Kleberschicht die Luft- und Wasserdampfdurchlässigkeit sich entsprechend verringert sowie der Klebemassenverbrauch steigt und bei geringer Flächendeckung der Kleberschicht die Klebeeigenschaften leiden, d.h., das Produkt löst sich, insbesondere bei schweren, textilen Trägermaterialien, zu leicht vom Untergrund, insbesondere von der Haut.

Bei den genannten druckempfindlichen Selbstklebemassen können die Massen für die Verarbeitung in einer Trägermatrix vorliegen. Als Trägermatrix werden gängige organische oder anorganische Lösemittel oder Dispergiermittel verstanden.

Systeme ohne Trägermatrix werden als 100 %-Systeme bezeichnet und sind ebenfalls nicht unbekannt. Sie werden im elastischen oder thermoplastischen Zustand verarbeitet. Ein gängiger Verarbeitungszustand ist die Schmelze.
Auch solche Haftschmelzklebemassen sind im Stande der Technik bereits vorbeschrieben. Sie basieren auf natürlichen oder synthetischen Kautschuken und/oder anderen synthetischen Polymeren.

Vorteilhaft an den 100 %-Systemen ist, daß bei ihnen verfahrenstechnisch ein Entfernen der Trägermatrix, d.h. der Hilfsmittel, vermieden wird, wodurch sich die Verarbeitungsproduktivität steigert und sich gleichzeitig der Maschinen- und Energieaufwand reduziert. Weiter wird so ein Verbleiben von Reststoffen der Trägermatrix reduziert. Dieses begünstigt wiederum die Senkung des allergenen Potentials.

Aufgabe der Erfindung war es, ein zumindest einseitig partiell selbstklebendes Trägermaterial zur Verfügung zu stellen, das aufgrund seiner Ausrüstung, also der Auftragsform und Eigenschaften der Klebemasse, sowie der Materialeigenschaften des Trägermaterials zur funktionsgerechten Verwendung für diverse Fixierungen, insbesondere für medizinische Produkte, dient und dabei sowohl funktionale wie auch wirtschaftliche Vorteile bietet.

Gelöst wird diese Aufgabe durch ein zumindest einseitig partiell selbstklebendes Trägermaterial, wie es im Hauptanspruch niedergelegt ist. Gegenstand der Untersprüche sind vorteilhafte Ausführungsformen des Trägermaterials, Verfahren zur Herstellung des Trägermaterials sowie besonders vorteilhafte Verwendungen.

Demgemäß betrifft die Erfindung ein zumindest einseitig partiell selbstklebend ausgerüstetes Trägermaterial, wobei der Auftrag der Selbstklebemasse in Form geometrischer Körper erfolgt. Zumindest bei einem Teil der geometrischen Körper liegt die Basisfläche, mit der die geometrischen Körper auf dem Trägermaterial haften, innerhalb der Projektionsfläche der geometrischen Körper, die sich daraus ergibt, daß die geometrischen Körper lotrecht auf das Trägermaterial projiziert werden.

Innerhalb der geometrischen Körper gibt es also zumindest eine Querschnittsfläche, die parallel zum Trägermaterial ausgerichtet ist und die größer ist als die Basisfläche, also der Fläche, mit der die geometrischen Körper auf dem Trägermaterial verankert sind.

Als Trägermaterialien eignen sich alle starren und elastischen Flächengebilde aus synthetischen und natürlichen Rohstoffen. Bevorzugt sind Trägermaterialien, die nach Applikation der Klebemasse so eingesetzt werden können, daß sie Eigenschaften eines funktionsgerechten Verbandes erfüllen. Beispielhaft sind Textilien wie Gewebe, Gewirke, Gelege, Vliese, Laminate, Netze, Folien, Schäume und Papiere aufgeführt. Weiter können diese Materialien vor- beziehungsweise nachbehandelt werden. Gängige Vorbehandlungen sind Corona und Hydrophobieren; geläufige Nachbehandlungen sind Kalandern, Tempern, Kaschieren, Stanzen und Eindecken.

Für die Beschichtung des Trägermaterials lassen sich vorteilhafterweise thermoplastische Heißschmelzklebemassen einsetzen auf Basis natürlicher und synthetischer Kautschuke und anderer synthetischer Polymere wie Acrylate, Methacrylate, Polyurethane, Polyolefine, Polyvinylderivate, Polyester oder Silikone mit entsprechenden Zusatzstoffen wie Klebharzen, Weichmachern, Stabilisatoren und anderen Hilfsstoffen soweit erforderlich.

Ihr Erweichungspunkt sollte höher als 50 °C liegen, da die Applikationstemperatur in der Regel mindestens 90 °C beträgt, bevorzugt zwischen 120 °C und 150 °C beziehungsweise 180 °C und 220 °C bei Silikonen. Gegebenenfalls kann eine Nachvernetzung durch UV- oder Elektronenstrahlen-Bestrahlung angebracht sein, um besonders vorteilhafte Eigenschaften der Heißschmelzklebemassen einzustellen.

Insbesondere Heißschmelzklebemassen auf Basis von Blockcopolymeren zeichnen sich durch ihre vielfältige Variationsmöglichkeiten aus, denn durch die gezielte Absenkung der Glasübergangstemperatur der Selbstklebemasse infolge der Auswahl der Klebrigmacher, der Weichmacher sowie der Polymermolekülgröße und der Molekularverteilung der Einsatzkomponenten wird die notwendige funktionsgerechte Verklebung mit der Haut auch an kritischen Stellen des menschlichen Bewegungsapparates gewährleistet.

Die hohe Scherfestigkeit der Heißschmelzklebemasse wird durch die hohe Kohäsivität des Polymeren erreicht. Die gute Anfaßklebrigkeit ergibt sich durch die eingesetzte Palette an Klebrigmachern und Weichmachern.

Für besonders starkklebende Systeme basiert die Heißschmelzklebemasse bevorzugt auf Blockcopolymeren, insbesondere A-B-, A-B-A-Blockcopolymere oder deren Mischungen. Die harte Phase A ist vornehmlich Polystyrol oder dessen Derivate, und die weiche Phase B enthält Ethylen, Propylen, Butylen, Butadien, Isopren oder deren Mischungen, hierbei besonders bevorzugt Ethylen und Butylen oder deren Mischungen.

Polystyrolblöcke können aber auch in der weichen Phase B enthalten sein, und zwar bis zu 20 Gew.-%. Der gesamte Styrolanteil sollte aber stets niedriger als 35 Gew.-% liegen. Bevorzugt werden Styrolanteile zwischen 5% und 30%, da ein niedrigerer Styrolanteil die Klebemasse anschmiegsamer macht.

Insbesondere die gezielte Abmischung von Di-Block- und Tri-Blockcopolymeren ist vorteilhaft, wobei ein Anteil an Di-Blockcopolymeren von kleiner 80 Gew.-% bevorzugt wird.

In einer vorteilhaften Ausführung weist die Heißschmelzklebemasse die nachfolgend angegebene Zusammensetzung auf:

| 10 Gew.-% bis 90 Gew.-% | Blockcopolymere, |
| --- | --- |
| 5 Gew.-% bis 80 Gew.-% | Klebrigmacher wie Öle, Wachse, Harze und/oder deren Mischungen, bevorzugt Mischungen aus Harzen und Ölen, |
| weniger als 60 Gew.-% | Weichmacher, |
| weniger als 15 Gew.-% | Additive, |
| weniger als 5 Gew.-% | Stabilisatoren. |

Die als Klebrigmacher dienenden aliphatischen oder aromatischen Öle, Wachse und Harze sind bevorzugt Kohlenwasserstofföle, -wachse und -harze, wobei sich die Öle, wie Paraffinkohlenwasserstofföle, oder die Wachse, wie Paraffinkohlenwasserstoffwachse, durch ihre Konsistenz günstig auf die Hautverklebung auswirken. Als Weichmacher finden mittel- oder langkettige Fettsäuren und/oder deren Ester Verwendung. Diese Zusätze dienen dabei der Einstellung der Klebeeigenschaften und der Stabilität. Gegebenenfalls kommen weitere Stabilisatoren und andere Hilfsstoffe zum Einsatz.

Ein Füllen der Klebemasse mit mineralischen Füllstoffen, Fasern, Mikrohohl- oder -vollkugeln ist möglich.

Die Heißschmelzklebemasse weist einen Erweichungspunkt auf oberhalb von 70 °C, bevorzugt 95 °C bis 120 °C.

Insbesondere an medizinische Trägermaterialien werden hohe Anforderungen bezüglich der Klebeeigenschaften gestellt. Für eine ideale Anwendung sollte die Heißschmelzklebemasse eine hohe Anfaßklebrigkeit besitzen. Die funktionsangepaßte Klebkraft auf der Haut und auf der Trägerrückseite sollte vorhanden sein. Weiterhin ist, damit es zu keinem Verrutschen kommt, eine hohe Scherfestigkeit der Heißschmelzklebemasse notwendig.

Durch die gezielte Absenkung der Glasübergangstemperatur der Klebemasse infolge der Auswahl der Klebrigmacher, der Weichmacher sowie der Polymermolekülgröße und der Molekularverteilung der Einsatzkomponenten wird die notwendige funktionsgerechte Verklebung mit der Haut und der Trägerrückseite erreicht.

Die hohe Scherfestigkeit der hier eingesetzten Klebemasse wird durch die hohe Kohäsivität des Blockcopolymeren erreicht. Die gute Anfaßklebrigkeit ergibt sich durch die eingesetzte Palette an Klebrigmachern und Weichmachern.

Die Produkteigenschaften wie Anfaßklebrigkeit, Glasübergangstemperatur und Scherstabilität lassen sich mit Hilfe einer dynamisch-mechanischen Frequenzmessung gut quantifizieren. Hierbei wird ein schubspannungsgesteuertes Rheometer verwendet.

Die Ergebnisse dieser Meßmethode geben Auskunft über die physikalischen Eigenschaften eines Stoffes durch die Berücksichtigung des viskoelastischen Anteils. Hierbei wird bei einer vorgegebenen Temperatur die Heißschmelzselbstklebemasse zwischen zwei planparallelen Platten mit variablen Frequenzen und geringer Verformung (linear viskoelastischer Bereich) in Schwingungen versetzt. Über eine Aufnahmesteuerung wird computerunterstützt der Quotient ($Q = \tan \delta$) zwischen dem Verlustmodul (G" viskoser Anteil) und dem Speichermodul (G' elastischer Anteil) ermittelt.

$$Q = \tan \delta = G''/G'$$

Für das subjektive Empfinden der Anfaßklebrigkeit (Tack) wird eine hohe Frequenz gewählt sowie für die Scherfestigkeit eine niedrige Frequenz.

Eine hoher Zahlenwert bedeutet eine bessere Anfaßklebrigkeit und eine schlechtere Scherstabilität.

Die Glasübergangstemperatur ist die Temperatur, bei der amorphe oder teilkristalline Polymere vom flüssigen oder gummielastischen Zustand in den hartelastischen oder glasigen Zustand übergehen oder umgekehrt (Römpp Chemie-Lexikon, 9. Aufl., Band 2, Seite 1587, Georg Thieme Verlag Stuttgart - New York, 1990). Er entspricht dem Maximum der Temperaturfunktion bei vorgegebener Frequenz.

Besonders für medizinische Anwendungen ist ein relativ niedriger Glasübergangspunkt notwendig.

| Bezeichnung | $T_G$ niedrige Frequenz | Anschmiegsamkeit niedrige Frequenz/RT | Anfaßklebrigkeit hohe Frequenz/RT |
|---|---|---|---|
| Heißschmetzklebemasse A | -12 ± 2 °C | $\tan \delta = 0{,}32 \pm 0{,}03$ | $\tan \delta = 1{,}84 \pm 0{,}03$ |
| Heißschmelzklebemasse B | -9 ± 2 °C | $\tan \delta = 0{,}22 \pm 0{,}03$ | $\tan \delta = 1{,}00 \pm 0{,}03$ |

Die Heißschmelzklebemassen sind vorzugsweise so eingestellt, daß sie bei einer Frequenz von 0,1 rad/s eine dynamisch-komplexe Glasübergangstemperatur von weniger als 15 °C, bevorzugt von 0 °C bis -30 °C, ganz besonders bevorzugt von -3 °C bis -15 °C, aufweisen.

Bevorzugt werden erfindungsgemäß Heißschmelzklebemassen, bei denen das Verhältnis des viskosen Anteils zum elastischen Anteil bei einer Frequenz von 100 rad/s bei 25 °C größer 0,7, insbesondere zwischen 1,0 und 5,0, ist, oder Heißschmelzselbstklebemassen, bei denen das Verhältnis des viskosen Anteils zum elastischen Anteil bei einer Frequenz von 0,1 rad/s bei 25 °C kleiner 0,4 ist, bevorzugt zwischen 0,35 und 0,02, ganz besonders bevorzugt zwischen 0,3 und 0,1.

Vorteilhaft insbesondere für die Verwendung des Trägermaterials bei medizinischen Produkten ist der partielle Auftrag der Klebmasse auf dem Trägermaterial, beispielsweise durch Rasterdruck, Thermosiebdruck, Thermoflexodruck oder Tiefdruck, denn im Vollstrich klebend ausgerüstete Trägermaterialien können unter ungünstigen Voraussetzungen bei der Applikation mechanische Hautirritationen hervorrufen. Alternativ kann der Auftrag auch nach dem Düsenverfahren erfolgen.

In einer bevorzugten Ausführungsform ist die Selbstklebemasse in Form von polygeometrischen Kalotten auf den Träger aufgebracht, ganz besonders in Form von solchen Kalotten, bei denen das Verhältnis Durchmesser zu Höhe kleiner 5:1 ist.

Die Kalotten können unterschiedliche Formen aufweisen. Bevorzugt sind abgeflachte Halbkugeln. Weiterhin ist auch der Aufdruck anderer Formen und Muster auf dem Trägermaterial möglich, so beispielsweise ein Druckbild in Form alphanumerischer Zeichenkombinationen oder Muster wie Gitter, Streifen, des weiteren Kumulate der Kalotten und Zickzacklinien.

Femer kann sie beispielsweise auch aufgesprüht sein, was ein mehr oder weniger unregelmäßiges Auftragsbild ergibt. Die Klebemasse kann gleichmäßig auf dem Trägermaterial verteilt sein, sie kann aber auch funktionsgerecht für das Produkt über die Fläche unterschiedlich stark oder dicht aufgetragen sein.

Durch die erfindungsgemäße Geometrie der Kalotten wird im Vergleich zu einem herkömmlichen partiell beschichteten Trägermaterial bei gleichbleibender Kontaktfläche zum Untergrund Klebemasse eingespart beziehungsweise bei gleichem Klebemassenauftragsgewicht eine höhere Haftung am Untergrund erreicht.

Dabei wird die für spezielle Anwendungen, insbesondere im medikalen Bereich, vorteilhafte hohe Durchlässigkeit der Klebeschicht für Luft und Wasserdampf nicht wesentlich beeinträchtigt.

Das bevorzugte Verfahren zur Herstellung eines selbstklebend ausgerüsteten Trägermaterials zeichnet sich durch insgesamt drei Verfahrensschritte aus.

Im ersten Schritt werden die aus Heißschmelzklebemasse gebildeten, geometrischen Körper durch Rasterdruck, Thermosiebdruck oder Tiefdruck oder durch das Düsenverfahren auf einen Hilfsträger aufgebracht, im zweiten Schritt der Hilfsträger mit den Körpern an das Trägermaterial geführt, schließlich die Körper vom Hilfsträger auf das Trägermaterial übertragen.

Unter dem Begriff Hilfsträger sollen herkömmliche, endlose Bahnen aus unterschiedlichen Materialien, aber auch Vorrichtungen wie Walzen verstanden werden.

Je nach Trägermaterial und dessen Temperaturempfindlichkeit kann aber die Heißschmelzklebemasse direkt auf das Trägermaterial aufgetragen werden.

Auch ein nachträgliches Kalandern des beschichteten Produktes und/oder eine Vorbehandlung des Trägers, wie Coronabestrahlung, zur besseren Verankerung der Klebeschicht kann vorteilhaft sein.

Weiterhin kann eine Behandlung der Heißschmelzklebemasse mit einer Elektronenstrahl-Nachvernetzung oder einer UV-Bestrahlung zu einer Verbesserung der gewünschten Eigenschaften führen.

Das Prinzip des Thermosiebdrucks besteht in der Verwendung einer rotierenden beheizten, nahtlosen, trommelförmigen perforierten Rundschablone, die über eine Düse mit der bevorzugten Heißschmelzklebemasse beschickt wird. Eine speziell geformte Düsenlippe (Rund- oder Vierkantrakel) preßt die über einen Kanal eingespeiste Heißschmelzklebemasse durch die Perforation der Schablonenwand auf die vorbeigeführte Trägerbahn. Diese wird mit einer Geschwindigkeit, die der Umgangsgeschwindigkeit der rotierenden Siebtrommel entspricht, mittels einer Gegendruckwalze gegen den Außenmantel der beheizten Siebtrommel geführt.

Die Ausbildung der kleinen Klebstoffkalotten geschieht dabei nach folgendem Mechanismus:

Der Düsenrakeldruck fördert die Heißschmelzklebemasse durch die Siebperforation an das Trägermaterial. Die Größe der ausgebildeten Kalotten wird durch den Durchmesser des Siebloches vorgegeben. Entsprechend der Transportgeschwindigkeit der Trägerbahn (Rotationsgeschwindigkeit der Siebtrommel) wird das Sieb vom Träger abgehoben. Bedingt durch die hohe Adhäsion der Klebemasse und die innere Kohäsion des Hotmelts wird von der auf dem Träger bereits haftenden Basis der Kalotten der in den Löchern begrenzte Vorrat an Heißschmelzklebemasse konturenscharf abgezogen beziehungsweise durch den Rakeldruck auf den Träger gefördert.

Nach Beendigung dieses Transportes formt sich, abhängig von der Rheologie der Heißschmelzklebemasse, über der vorgegebenen Basisfläche die mehr oder weniger stark gekrümmte Oberfläche der Kalotte. Das Verhältnis Höhe zur Basis der Kalotte hängt vom Verhältnis Lochdurchmesser zur Wandstärke der Siebtrommel und den physikalischen Eigenschaften (Fließverhalten, Oberflächenspannung und Benetzungswinkel auf dem Trägermaterial) der Selbstklebemasse ab.

Bei der Siebschablone im Thermosiebdruck kann das Steg/Loch-Verhältnis kleiner 10:1 sein, bevorzugt kleiner oder gleich 1:1, insbesondere gleich 1:5.

Der beschriebene Bildungsmechanismus der Kalotten erfordert bevorzugt saugfähige oder zumindest von Heißschmelzklebemasse benetzbare Trägermaterialien. Nichtbenetzende Trägeroberflächen müssen durch chemische oder physikalische Verfahren vorbehandelt werden. Dies kann durch zusätzliche Maßnahmen wie zum Beispiel Coronaentladung oder Beschichtung mit die Benetzung verbessernden Stoffen geschehen.

Mit dem aufgezeigten Druckverfahren kann die Größe und Form der Kalotten definiert festgelegt werden. Die für die Anwendung relevanten Klebkraftwerte, die die Qualität der erzeugten Produkte bestimmen, liegen bei sachgerechter Beschichtung in sehr engen Toleranzen. Der Basisdurchmesser der Kalotten kann von 10 µm bis 5000 µm gewählt werden, die Höhe der Kalotten von 20 µm bis 2000 µm, bevorzugt 50 µm bis 1000 µm, wobei der Bereich kleiner Durchmesser für glatte Träger, der mit größerem

Durchmesser und größerer Kalottenhöhe für rauhe oder stark porige Trägermaterialien vorgesehen ist.

Die Positionierung der Kalotten auf dem Träger wird durch die in weiten Grenzen variierbare Geometrie des Auftragswerkes, zum Beispiel Gravur- oder Siebgeometrie, definiert festgelegt. Mit Hilfe der aufgezeigten Parameter kann über einstellbare Größen das gewünschte Eigenschaftsprofil der Beschichtung, abgestimmt auf die verschiedenen Trägermaterialien und Anwendungen, sehr genau eingestellt werden.

Das Trägermaterial wird bevorzugt mit einer Geschwindigkeit von größer 2 m/min, bevorzugt 20 bis 200 m/min, beschichtet, wobei die Beschichtungstemperatur größer als die Erweichungstemperatur zu wählen ist.

Der prozentuale Anteil, der mit der Heißschmelzklebemasse beschichteten Fläche sollte mindestens 20 % betragen und kann bis zu ungefähr 95 % reichen, für spezielle Produkte bevorzugt 40 % bis 60 % sowie 70 % bis 95 %. Dieses kann gegebenenfalls durch Mehrfachapplikation erreicht werden, wobei gegebenenfalls auch Heißschmelzklebemassen mit unterschiedlichen Eigenschaften eingesetzt werden können.

Durch eine gesteuerte Temperaturführung und/oder das Einbringen von Strahlungs-, mechanischer oder Sekundärenergie während der Herstellung kann bei thermoplastischen Selbstklebemassen eine Geometriemodifizierung der geometrischen Körper erfolgen, wobei die Kopf- und Basisdurchmesser in großen Grenzen variiert werden können. Bevorzugt kann auch eine gesteuerte Temperaturführung an der Oberfläche der thermoplastischen oder elastischen Primärkalotten, also der auf dem Hilfsträger aufgebrachten Kalotten, erfolgen, zum Beispiel durch Strahlungserhitzung wie IR, mit deren Hilfe Sekundärkalotten erzeugt werden können, die für eine Produkttransferierung optimale Verklebungseigenschaften aufweisen.

Eine Addition bei der Energiesteuerung im Material und/oder an der Oberfläche der Polymere läßt eine große Vielfalt von polygeometrischen Kalottenformen zu. Zur endgültigen Ausformung der transferierten Sekundärkalotte kann eine regulierbare Druckstation (Spart/Druck/Temperatur/Geschwindigkeit) von Vorteil sein. Auch nachträgliches Kalandern kann vorteilhaft sein.

Das vor der Transferierung der Sekundärkalotte vorliegende viskoelastische Eigenschaftsprofil der Körper aus Selbstklebemasse kann durch das Steuern der Wärmeenergie aus dem Beschichtungsprozeß, das wenigstens teilweise Einbringen von Oberflächenenergie oder den wenigstens teilweisen Entzug von Wärmeenergie oder eine Kombination der Verfahren eingestellt werden.

Die geometrischen Körper lassen sich demgemäß in mehrere Zonen einteilen, die durchaus unterschiedliche Eigenschaften aufweisen können. Vorzugsweise haben die auf den Hilfsträger aufgebrachten geometrischen Körper zum Zeitpunkt des Transfers auf das Trägermaterial in der Basiszone, die dem Teil des Körpers entspricht, der sich am Trägermaterial anschließt, ein Plastizitäts-/Elastizitätsverhältnis bei einer Frequenz von 100 rad/s von 0,4 bis 50 und in der Kopfzone, die der Basiszone gegenüberliegend den äußeren Teil des Körpers darstellt, ein Plastizitäts-/Elastizitätsverhältnis von größer 0,3, bevorzugt 0,4 bis 50, wobei das Plastizitäts-/Elastizitätsverhältnis in der Kopfzone nicht kleiner ist als in der Basiszone.

Des weiteren hat es sich als vorteilhaft erwiesen, wenn das Höhenverhältnis von Kopfzone zu Basiszone zwischen 5 und 95%, bevorzugt zwischen 10 und 50 % eingestellt ist.

Die Kombination der Heißschmelzklebemasse und der partiellen Beschichtung sichert eine sichere Verklebung des Trägermaterials.

Insbesondere wenn das Trägermaterial zur Herstellung eines medizinischen Produktes verwendet wird, haftet dieses auf der Haut, auf der anderen Seite sind zumindest visuell erkennbare allergische oder mechanische Hautirritationen ausgeschlossen, auch bei einer Anwendung, die sich über mehrere Tage erstreckt.

Der partielle Auftrag ermöglicht durch geregelte Kanäle die Abführung des transepidermalen Wasserverlustes und verbessert das Ausdampfen der Haut beim Schwitzen insbesondere bei der Verwendung von luft- und wasserdampfdurchlässigen Trägermaterilien. Hierdurch werden Hautirritationen, die durch Stauungen der Körperflüssigkeiten hervorgerufen werden, vermieden. Die angelegten Abführungskanäle ermöglichen ein Ableiten auch unter Verwendung eines mehrlagigen Verbandes.

Die Epilation entsprechender Körperregionen und der Massetransfer auf die Haut sind aufgrund der hohen Kohäsivität des Klebers vernachlässigbar, weil der Kleber nicht an Haut und Haar verankert, vielmehr ist die Verankerung der Klebemasse auf dem Trägermaterial mit bis zu 12 N/cm (Probenbreite) für medizinische Anwendungen gut.

Durch die ausgeformten Sollbruchstellen in der Beschichtung werden Hautlagen beim Ablösen nicht mehr mit- oder gegeneinander verschoben. Das Nichtverschieben der Hautlagen und die geringere Epilation führen zu einem bisher nicht gekannten Grad der Schmerzfreiheit bei solchen stark klebenden Systemen. Weiter unterstützt die individuelle biomechanische Klebkraftsteuerung, welche eine nachweisliche Absenkung der Klebkraft dieser Pflaster aufweist, die Ablösbarkeit. Das applizierte Trägermaterial zeigt gute propriorezeptive Wirkungen.

In einer weiteren vorteilhaften Ausführungsform werden die Selbstklebemassen geschäumt, bevor sie auf das Trägermaterial aufgetragen werden.

Die Selbstklebemassen werden dabei bevorzugt mit inerten Gasen wie Stickstoff, Kohlendioxid, Edelgasen, Kohlenwasserstoffen oder Luft oder deren Gemischen geschäumt. In manchen Fällen hat sich ein Aufschäumen zusätzlich durch thermische Zersetzung gasentwickelnder Substanzen wie Azo-, Carbonat- und Hydrazid-Verbindungen als geeignet erwiesen.

Der Schäumungsgrad, d.h. der Gasanteil, sollte mindestens etwa 5 Vol.-% betragen und kann bis zu etwa 85 Vol.-% reichen. In der Praxis haben sich Werte von 10 Vol.-% bis 75 Vol.-%, bevorzugt 50 Vol.-%, gut bewährt. Wird bei relativ hohen Temperaturen von ungefähr 100 °C und vergleichsweise hohem Innendruck gearbeitet, entstehen sehr offenporige Klebstoffschaumschichten, die besonders gut luft- und wasserdampfdurchlässig sind.

Die vorteilhaften Eigenschaften der geschäumten Selbstklebebeschichtungen wie geringer Klebstoffverbrauch, hohe Anfaßklebrigkeit und gute Anschmiegsamkeit auch an unebenen Flächen durch die Elastizität und Plastizität sowie der Initialtack lassen ganz besonders sich auf dem Gebiet der medizinischen Produkte optimal nutzen.

Durch den Einsatz von atmungsaktiven Beschichtungen in Verbindung mit elastischen ebenfalls atmungsaktiven Trägermaterialien ergibt sich ein vom Anwender subjektiv angenehmer empfundener Tragekomfort.

Ein besonders geeignetes Verfahren zur Herstellung der geschäumten Selbstklebemasse arbeitet nach dem Schaum-Mix-System. Hierbei wird die thermoplastische Selbstklebemasse unter hohem Druck bei einer Temperatur über dem Erweichungspunkt (ungefähr 120 °C) mit den vorgesehenen Gasen wie zum Beispiel Stickstoff, Luft oder Kohlendioxid in unterschiedlichen Volumenanteilen (etwa 10 Vol.-% bis 80 Vol.-%) in einem Stator/Rotorsystem umgesetzt.

Während der Gasvordruck größer 100 bar ist, betragen die Mischdrucke Gas/Thermoplast im System 40 bis 100 bar, bevorzugt 40 bis 70 bar. Der so hergestellte Haftklebeschaum kann anschließend über eine Leitung in das Auftragswerk gelangen. Bei dem Auftragswerk finden handelsübliche Düsen, Extruder- oder Kammersysteme Verwendung.

Durch die Schäumung der Selbstklebemasse und die dadurch entstandenen offenen Poren in der Masse sind bei Verwendung eines an sich porösen Trägers die mit der Klebemasse beschichteten Produkte gut wasserdampf- und luftdurchlässig. Die benötigte Klebemassenmenge wird erheblich reduziert ohne Beeinträchtigung der Klebeeigenschaften. Die Klebemassen weisen eine überraschend hohe Anfaßklebrigkeit (tack) auf, da pro Gramm Masse mehr Volumen und damit Klebeoberfläche zum Benetzen des zu beklebenden Untergrundes zur Verfügung steht und die Plastizität der Klebemassen durch die Schaumstruktur erhöht ist. Auch die Verankerung auf dem Trägermaterial wird dadurch verbessert. Außerdem verleiht die geschäumte Klebebeschichtung, wie bereits oben erwähnt, den Produkten ein weiches und anschmiegsames Anfühlen.

Durch das Schäumen wird zudem die Viskosität der Klebemassen in der Regel gesenkt. Hierdurch wird die Schmelzenergie erniedrigt, und es können auch thermoinstabile Trägermaterialien direkt beschichtet werden.

Die Heißschmelzklebemasse kann mit einem Flächengewicht von größer 6 g/m$^2$, bevorzugt zwischen 20 g/m$^2$ und 300 g/m$^2$, ganz besonders bevorzugt zwischen 40 g/m$^2$ und 180 g/m$^2$, auf dem Trägermaterial aufgetragen sein.

Das mit der Klebemasse beschichtete Trägermaterial kann eine Luftdurchlässigkeit von größer 1 cm$^3$/(cm$^2$*s), bevorzugt größer 15 cm$^3$/(cm$^2$*s), ganz besonders bevorzugt größer 70 cm$^3$/(cm$^2$*s), aufweisen, des weiteren eine Wasserdampfdurchlässigkeit von größer 200 g/(m$^2$*24h), bevorzugt größer 500 g/(m$^2$*24h), ganz besonders bevorzugt größer 2000 g/(m$^2$*24h).

Das erfindungsgemäße Trägermaterial weist eine Klebkraft auf der Trägerrückseite von mindestens 0,5 N/cm auf, besonders eine Klebkraft zwischen 2,5 N/cm und 5 N/cm. Auf anderen Untergründen können höhere Klebkräfte erreicht werden.

Die hervorragenden Eigenschaften des erfindungsgemäßen, selbstklebend ausgerüsteten Trägermaterials legen die Verwendung für medizinische Produkte, insbesondere Pflaster, medizinische Fixierungen, Wundabdeckungen, dotierte Systeme, orthopädische oder phlebologische Bandagen und Binden nahe. Bei den dotierten Systemen sind insbesondere solche bevorzugt, die Stoffe freisetzen.

Schließlich kann das Trägermaterial nach dem Beschichtungsvorgang mit einem klebstoffabweisenden Trägermaterial, wie silikonisiertes Papier, eingedeckt oder mit einer Wundauflage oder einer Polsterung versehen werden.

Besonders vorteilhaft ist, daß das Trägermaterial sterilisierbar, bevorzugt γ-(gamma) sterilisierbar, ist. So sind besonders geeignet für eine nachträgliche Sterilisation Heißschmelzklebemassen auf Blockcopolymerbasis, welche keine Doppelbindungen enthalten. Dieses gilt ins besondere für Styrol-Butylen-Ethylen-Styrol-Blockcopolymerisate oder Styrol-Butylen-Styrol-Blockcopolymerisate. Es treten hierbei keine für die Anwendung signifikanten Änderungen in den Klebeeigenschaften auf.

Es eignet sich auch hervorragend für technische reversible Fixierungen, welche beim Abziehen keine Verletzung oder Beschädigung von diversen Untergründen, wie Papier, Kunststoffe, Glas, Textilien, Holz, Metalle oder Mineralien, zulassen.

Schließlich können technisch permanente Verklebungen hergestellt werden, welche nur unter teilweiser Spaltung des Untergrundes getrennt werden können.

Anhand mehrerer Figuren sollen vorteilhafte Ausführungsformen des Erfindungsgegenstandes dargestellt werden, ohne damit die Erfindung unnötig beschränken zu wollen.

Es zeigen

Figur 1    ein mit halbkugelförmigen Kalotten beschichtetes Trägermaterial,

Figur 2    das beschichtete Trägermaterial gemäß Figur 1 in der Draufsicht und

Figur 3    ein mit unterschiedlich geformten Kalotten im seitlichen Schnitt beschichtetes Trägermaterial.

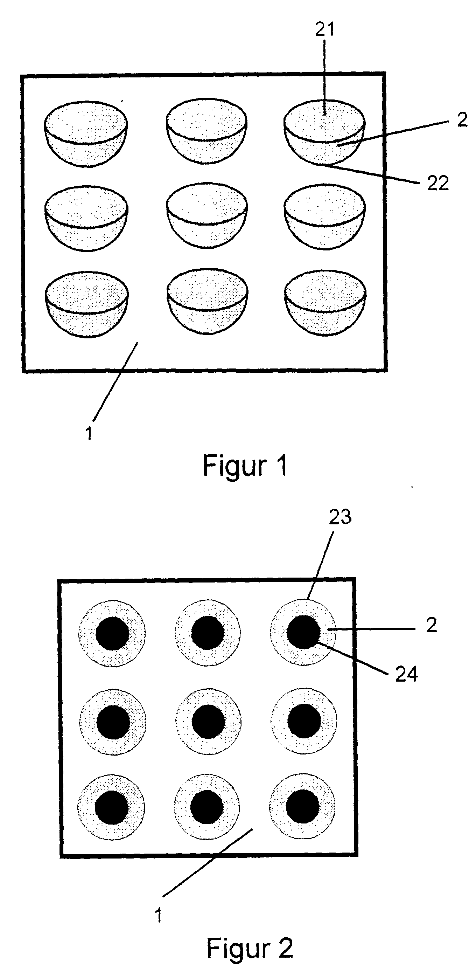

Die Figur 1 zeigt einen Ausschnitt aus einem endlosen Trägermaterial 1, das mit im wesentlichen halbkugelförmigen Kalotten 2 beschichtet ist. In jeder einzelnen Kalotte 2 lassen sich eine Basiszone 22, die in dem Bereich der Kalotte 2 liegt, in dem die Kalotte 2 auf dem Trägermaterial 1 verankert ist, sowie die der Basiszone 22 gegenüberliegende Kopfzone 21 unterscheiden. Beide Zonen 21, 22 können gegebenenfalls voneinander abweichende Eigenschaften aufweisen.

Die Figur 2, die das beschichtete Trägermaterial gemäß Figur 1 in der Draufsicht zeigt, dient zur Verdeutlichung der Basisfläche 24 sowie der Projektionsfläche 23. Die Basisfläche 24 ist diejenige Fläche der Kalotte 2, mit der die Kalotte auf dem Trägermaterial 1 verankert ist. Projektionsfläche 23 ist die Fläche, die sich bei der lotrechten Projektion der Kalotte 2 auf das Trägermaterial 1 ergibt. Erfindungsgemäß sind die Kalotten 2 stets so geformt, daß die Basisfläche 24 innerhalb der Projektionsfläche 23 liegt.

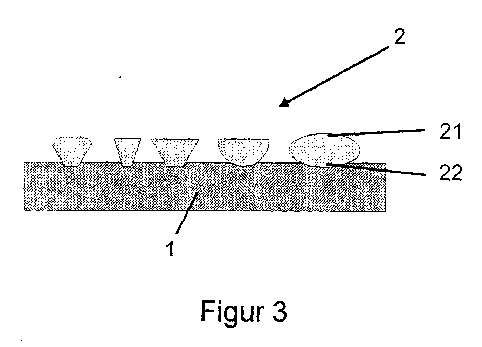

In der Figur 3 sind schließlich unterschiedlich geformte Kalotten 2 im seitlichen Schnitt dargestellt, die sich als besonders vorteilhaft erwiesen haben. Besonders deutlich lassen sich an der rechten Kalotte nochmals die Basiszone 22 und die Kopfzone 21 darstellen.

Im folgenden soll ein erfindungsgemäßes selbstklebend ausgerüstetes Trägermaterial mittels eines Beispiels dargestellt werden, ohne auch hier die Erfindung unnötig einschränken zu wollen.

**Beispiel 1**

Erfindungsgemäß wurde eine unelastische selbstklebende Binde hergestellt, die aufgrund ihrer nachfolgend beschriebenen Eigenschaften zur Anwendung als funktioneller Tapeverband dienen kann, wobei sich die Funktionelle Verbandtechnik an der Anatomie und der Biomechanik orientiert.

Die für diesen Verbandtyp verwendete Binde bestand aus einem unelastischen Baumwollgewebe mit einer Höchstzugkraft von größer 60 N/cm und einer HöchstzugkraftDehnung von kleiner 20 %.

Die Selbstklebemasse wurde im Thermosiebdruck auf den Träger appliziert, wobei es sich bei der Selbstklebemasse um einen Heißschmelzkleber handelte.

Diese Schmelzklebemasse setzte sich wie folgt zusammen:

-   ein A-B/A-B-A Blockcopolymer, welches aus harten und weichen Segmenten besteht, mit einem Verhältnis der A-B-A zur A-B von 2:1 und einem Styrolgehalt im Polymer von 13 Mol.-%; der Anteil an der Klebemasse beträgt 40 Gew.-% (Kraton G)
-   ein Paraffinkohlenwasserstoffwachs mit einem Anteil an der Klebmasse von 45 Gew.-%
-   Kohlenwasserstoffharze mit einem Anteil von 14,5 Gew.-% (Super Resin HC 140)
-   ein Alterungsschutzmittel mit einem Anteil von weniger als 0,5 Gew.-% (Irganox)

Die eingesetzten Komponenten wurden in einem Thermomischer bei 195 °C homogenisiert.

Der Erweichungspunkt dieser Klebemasse betrug ca. 85 °C (DIN 52011), und die Klebmasse zeigte eine Viskosität von 2100 mPas bei 150 °C (DIN 53018, Brookfield DV II, Sp. 21). Die Glasübergang betrug nach oben dargelegten Methode -7°C.

Die erfindungsgemäße Haftklebergeometrie wurde mittels des beschriebenen Transfers der Haftklebemassenkalotten von einem Hilfsträger auf das Trägermaterial realisiert:

Die indirekte Beschichtung erfolgte mit 50 m/min bei einer Temperatur von 120 °C auf eine vorbehandelte Gegendruckwalze (Hilfsträger) und von dort auf das Trägermaterial.

Kurz bevor das Trägermaterial zugeführt wurde, wurde mit Strahlung der Klebemasse Energie zugeführt. Diese führte zu einer Aufteilung der auf dem Hilfsträger haftenden Klebemassenkalotte in eine Kopfzone mit hoher Plastizität und niedriger Elastizität sowie in eine Basiszone mit geringer Plastizität und hoher Elastizität. Dazwischen stellte sich eine Übergangszone ein.

Beim druckbeaufschlagten Transfer auf das textile Trägermaterial dran die niedrigviskose Kopfzone leicht in das Trägermaterial ein und sorgte dort für eine gute Verankerung der Klebemassenkalotte; die hochviskose Basiszone legte sich auf das Trägermaterial und verhinderte ein übermäßiges Eindringen des Haftklebstoffes in das Trägermaterial.

Als vorteilhaft hat es sich erwiesen, die gesamten Haftschmelzkleberkalotten bis zum Transfer in den gewünschten viskoelastischen Zustand der Basiszone zu bringen beziehungsweise diesen aus der Schmelzwärme zu erhalten und durch einen kurzen Strahlungsimpuls unmittelbar vor dem Transfer lediglich die Kopfzone in den gewünschten niedrigviskosen Zustand zu bringen.

Das Trägermaterial wurde mit 120 g/m$^2$ beschichtet, wobei eine 14 HX-Meshsieb-Schablone verwendet wurde.

Die Punktbasis war in seiner Fläche 5 % kleiner als der Punktkopf. Hierdurch wurde ein stabileres Verkleben der Punkte erreicht, da eine Kantenecke auf der Verklebungsfläche des haftklebrigen Flächengebildes fehlte.

Durch den Transfer vom glatten Hilfsträger erhielt die Klebemassenoberfläche zudem eine ebene Oberfläche, die gleichfalls die geeignet war, die Kontaktfläche zu ebenen Untergründen zu vergrößern.

Gegenüber einer Vergleichsprobe mit gleichem Masseauftrag war die Klebkraft auf der Trägerrückseite um 15 % höher.

Die nach diesem Verfahren hergestellte Binde zeigte ein reversibles Ablösen von der Haut sowie eine gute Luft- und Wasserdampfdurchlässigkeit. Aufgrund der hohen Scherstabilität des Schmelzhaftklebers wurde eine ausreichend Stabilisierung und eine gute propriorezeptive Wirkung festgestellt. Es wurden keine Hautirritationen und eine vernachlässigbar geringe Epilation nach dem Abnehmen der Binde beobachtet.

**Patentansprüche**

1. Zumindest einseitig partiell selbstklebend ausgerüstetes Trägermaterial, wobei der Auftrag der Selbstklebemasse in Form geometrischer Körper erfolgt, **dadurch gekennzeichnet, daß** zumindest bei einem Teil der geometrischen Körper die Basisfläche, mit der die geometrischen Körper auf dem Trägermaterial haften, innerhalb der Projektionsfläche der geometrischen Körper liegt, die sich daraus ergibt, daß die geometrischen Körper lotrecht auf das Trägermaterial projiziert werden.

2. Selbstklebend ausgerüstetes Trägermaterial gemäß Anspruch 1, **dadurch gekennzeichnet, daß** die Selbstklebemasse in Form von polygeometrischen Kalotten auf den Träger aufgebracht ist.

3. Trägermaterial gemäß Anspruch 1 oder 2, **dadurch gekennzeichnet, daß** die Selbstklebemasse eine Heißschmelzklebemasse ist.

4. Trägermaterial nach einem oder mehreren der vorhergehenden Ansprüche, **dadurch gekennzeichnet, daß** die Heißschmelzklebemasse auf Blockcopolymerbasis aufgebaut ist, insbesondere A-B- oder A-B-A-Blockcopolymere oder deren Mischungen, wobei Phase A vornehmlich Polystyrol oder dessen Derivate und Phase B Ethylen, Propylen, Butylen, Butadien, Isopren oder deren Mischungen, hierbei besonders bevorzugt Ethylen und Butylen oder deren Mischungen, sind.

5. Trägermaterial nach einem oder mehreren der vorhergehenden Ansprüche, **dadurch gekennzeichnet, daß** der Masseauftrag auf das Trägermaterial größer 6 $g/m^2$ beträgt, bevorzugt 20 bis 300 $g/m^2$, besonders bevorzugt 40 bis 180 $g/m^2$.

6. Trägermaterial nach einem oder mehreren der vorhergehenden Ansprüche, **dadurch gekennzeichnet, daß** das Trägermaterial aufweist eine Luftdurchlässigkeit von größer 1 $cm^3/(cm^2{*}s)$, bevorzugt 10 bis 150 $cm^3/(cm^2{*}s)$, und/oder eine Wasserdampfdurchlässigkeit von größer 200 $g/(m^2{*}24h)$, bevorzugt 500 bis 5000 $g/(m^2{*}24h)$.

7. Trägermaterial nach einem oder mehreren der vorhergehenden Ansprüche, **dadurch gekennzeichnet, daß** das Trägermaterial eine Klebkraft auf der Trägerrückseite von mindestens 0,5 N/cm aufweist, besonders eine Klebkraft zwischen 2,5 N/cm und 5 N/cm.

8. Verfahren zur Herstellung eines selbstklebend ausgerüsteten Trägermaterials gemäß einem oder mehreren der vorhergehenden Ansprüche, wobei

   a) die aus Heißschmelzklebemasse gebildeten, geometrischen Körper durch Rasterdruck, Thermosiebdruck oder Tiefdruck oder durch das Düsenverfahren auf einen Hilfsträger aufgebracht werden,
   b) der Hilfsträger mit den Körpern an das Trägermaterial geführt werden,
   c) die Körper vom Hilfsträger auf das Trägermaterial übertragen werden.

9. Verfahren gemäß Anspruch 8, **dadurch gekennzeichnet, daß** durch eine gesteuerte Temperaturführung und/oder das Einbringen von Strahlungs-, mechanischer oder Sekundärenergie eine Modifizierung der geometrischen Körper erfolgt.

10. Verfahren gemäß der Ansprüche 8 oder 9, **dadurch gekennzeichnet, daß** das viskoelastische Eigenschaftsprofil der geometrischen Körper durch das Steuern der Wärmeenergie aus dem Beschichtungsprozeß, das wenigstens teilweise Einbringen von Oberflächenenergie oder den wenigstens teilweisen Entzug von Wärmeenergie oder eine Kombination der Verfahren eingestellt wird.

**11.** Verfahren gemäß der Ansprüche 8 bis 10, **dadurch gekennzeichnet, daß** die auf den Hilfsträger aufgebrachten geometrischen Körper zum Zeitpunkt des Transfers auf das Trägermaterial in der Kopfzone ein Plastizitäts-/Elastizitätsverhältnis bei einer Frequenz von 100 rad/s von größer 0,3, bevorzugt 0,4 bis 50, und in der Basiszone ein Plastizitäts-/Elastizitätsverhältnis von 0,4 bis 50 aufweisen, wobei das Plastizitäts-/Elastizitätsverhältnis in der Kopfzone nicht kleiner ist als in der Basiszone.

**12.** Verfahren gemäß der Ansprüche 8 bis 11, **dadurch gekennzeichnet, daß** die Heißschmelzklebemasse aufgeschäumt ist.

**13.** Verfahren gemäß der Ansprüche 8 bis 12, **dadurch gekennzeichnet, daß** die Heißschmelzklebemasse elektronenstrahl- oder UV-vernetzbar ist.

**14.** Verwendung gemäß nach einem oder mehreren der vorhergehenden Ansprüche für medizinische Produkte, insbesondere Pflaster, medizinische Fixierungen, Wundabdeckungen, dotierte Systeme, orthopädische oder phlebologische Bandagen und Binden.

**15.** Verwendung gemäß Anspruch 14, **dadurch gekennzeichnet, daß** das selbstklebend ausgerüstete Trägermaterial nach der Applikation eingedeckt oder mit einer Wundauflage, Polsterung versehen wird.

**16.** Verwendung gemäß Anspruch 14, **dadurch gekennzeichnet, daß dadurch gekennzeichnet, daß** das selbstklebend ausgerüstete Trägermaterial sterilisierbar, bevorzugt γ (gamma)-sterilisierbar, ist.

**17.** Verwendung nach einem oder mehreren der vorhergehenden Ansprüche für technische reversible Fixierungen, welche beim Abziehen keine Verletzung oder Beschädigung von diversen Untergründen, wie Papier, Kunststoffe, Glas, Textilien, Holz, Metalle oder Mineralien, zulassen.

**18.** Verwendung nach einem oder mehreren der vorhergehenden Ansprüche für technisch permanente Verklebungen, welche nur unter teilweiser Spaltung des Untergrundes getrennt werden können.

**Claims**

**1.** Backing material with a partial self-adhesive coating on at least one side, the application of the self-adhesive composition taking place in the form of geometric structures, **characterized in that** for at least some of the geometric structures the base area by means of which the geometric structures adhere to the backing material lies within the area of projection of the geometric structures which is formed as a consequence of the fact that the geometric structures are projected perpendicularly onto the backing material.

**2.** Backing material with a self-adhesive coating according to Claim 1, **characterized in that** the self-adhesive composition has been applied in the form of polygeometric domes to the backing.

**3.** Backing material according to Claim 1 or 2, **characterized in that** the self-adhesive composition is a hotmelt adhesive composition.

**4.** Backing material according to one or more of the preceding claims, **characterized in that** the hotmelt adhesive composition is composed on the basis of block copolymers, especially A-B or A-B-A block copolymers or mixtures thereof, where phase A is principally polystyrene or its derivatives and phase B is ethylene, propylene, butylene, butadiene, isoprene or mixtures thereof, among which particular preference is given to ethylene and butylene or their mixtures.

**5.** Backing material according to one or more of the preceding claims, **characterized in that** the application of composition to the backing material is greater than 6 g/m$^2$, preferably from 20 to 300 g/m$^2$ and, with particular preference, from 40 to 180 g/m$^2$.

**6.** Backing material according to one or more of the preceding claims, **characterized in that** the backing material has an air permeability of greater than 1 cm$^3$/(cm$^2$*s), preferably from 10 to 150 cm$^3$/(cm$^2$*s), and/or a water vapour permeability of greater than 200 g/(m$^{2*}$24h), preferably from 500 to 5000 g/(m$^{2*}$24h).

7. Backing material according to one or more of the preceding claims, **characterized in that** the backing material has a bond strength to the reverse of the backing of at least 0.5 N/cm, in particular a bond strength of between 2.5 and 5 N/cm.

8. Process for producing a backing material with a self-adhesive coating according to one or more of the preceding claims, where

> a) the geometric structures formed from hotmelt adhesive composition are applied by halftone printing, thermal screen printing or intaglio printing, or by the nozzle method, to an auxiliary support,
> b) the auxiliary support with the structures is guided onto the backing material, and
> c) the structures are transferred from the auxiliary support to the backing material.

9. Process according to Claim 8, **characterized in that** the geometric structures are modified by a controlled temperature regime and/or by the introduction of radiative, mechanical or secondary energy.

10. Process according to Claim 8 or 9, **characterized in that** the profile of the viscoelastic properties of the geometric structures is adjusted by controlling the thermal energy from the coating process, by the at least partial introduction of surface energy or by the at least partial removal of thermal energy, or by a combination of the methods.

11. Process according to Claims 8 to 10, **characterized in that** the geometric structures applied to the auxiliary support, at the time of transfer onto the backing material, have - in the head zone - a plasticity/elasticity ratio at a frequency of 100 rad/s of greater than 0.3, preferably from 0.4 to 50, and - in the base zone - a plasticity/elasticity ratio of from 0.4 to 50, the plasticity/elasticity ratio in the head zone not being lower than that in the base zone.

12. Process according to Claims 8 to 11, **characterized in that** the hotmelt adhesive composition has been foamed.

13. Process according to Claims 8 to 12, **characterized in that** the hotmelt adhesive composition can be crosslinked by UV or electron beams

14. Use according to one or more of the preceding claims for medical products, especially plasters, medical fixings, wound covers, doped systems, orthopaedic or phlebological bandages and dressings.

15. Use according to Claim 14, **characterized in that** the backing material which has been given a self-adhesive treatment is lined following application or is provided with a wound pad or padding.

16. Use according to Claim 14, **characterized in that** the backing material which has been given a self-adhesive treatment is sterilizable, preferably by means of γ (gamma) radiation.

17. Use according to one or more of the preceding claims for technical reversible fixings which on removal cause no damage to a variety of substrates, such as paper, plastics, glass, textiles, wood, metals or minerals.

18. Use according to one or more of the preceding claims for technically permanent bonds which can be separated only with partial splitting of the substrate.

**Revendications**

1. Matériau support apprêté de manière auto-adhésive en discontinu sur au moins une face, dans lequel le dépôt de la masse auto-adhésive est effectué sous la forme de corps géométriques, **caractérisé en ce qu'**au moins dans une partie des corps géométriques, la face de base, par laquelle les corps géométriques adhèrent sur le matériau support, est située à l'intérieur de la face de projection des corps géométriques, qui est obtenue en effectuant la projection orthogonale des corps géométriques sur le matériau support.

2. Matériau support apprêté de manière auto-adhésive suivant la revendication 1, **caractérisé en ce que** la masse auto-adhésive est déposée sur le support sous la forme de calottes polygéométriques.

3. Matériau support suivant la revendication 1 ou 2, **caractérisé en ce que** la masse auto-adhésive est une masse adhésive thermofusible.

**4.** Matériau support suivant une ou plusieurs des revendications précédentes, **caractérisé en ce que** la masse adhésive thermofusible est élaborée à base de copolymères à blocs, en particulier de copolymères à blocs A-B ou A-B-A ou de leurs mélanges, dans lesquels la phase A est principalement du polystyrène ou ses dérivés et la phase B l'éthylène, le propylène, le butylène, le butadiène, l'isoprène ou leurs mélanges, en l'espèce de préférence particulièrement l'éthylène et le butylène ou leurs mélanges.

**5.** Matériau support suivant une ou plusieurs des revendications précédentes, **caractérisé en ce que** le dépôt massique sur le matériau support est supérieur à 6 g/m$^2$, de préférence de 20 à 300 g/m$^2$, et de préférence encore de 40 à 180 g/m$^2$.

**6.** Matériau support suivant une ou plusieurs des revendications précédentes, **caractérisé en ce que** le matériau support présente une perméabilité à l'air supérieure à 1 cm$^3$/(cm$^2$*s), de préférence de 10 à 150 cm$^3$/(cm$^2$*s), et/ ou une perméabilité à la vapeur d'eau supérieure à 200 g/(m$^2$*24h), de préférence de 500 à 5000 g/(m$^2$*24h).

**7.** Matériau support suivant une ou plusieurs des revendications précédentes, **caractérisé en ce que** le matériau support présente une force d'adhérence sur la face arrière du support d'au moins 0,5 N/cm, en particulier une force d'adhérence comprise entre 2,5 N/cm et 5 N/cm.

**8.** Procédé pour la fabrication d'un matériau support apprêté de manière auto-adhésive suivant une ou plusieurs des revendications précédentes, dans lequel

a) les corps géométriques formés à partir de la masse adhésive thermofusible sont déposés par impression en trame, thermosérigraphie ou en creux ou par le procédé d'impression par filière, sur un support auxiliaire,
b) le support auxiliaire avec les corps géométriques est conduit sur le matériau support,
c) les corps sont transférés du support auxiliaire sur le matériau support.

**9.** Procédé suivant la revendication 8, **caractérisé en ce que** l'on effectue une modification des corps géométriques par une conduite appropriée de la température et/ou par l'apport d'énergie rayonnante, mécanique ou secondaire.

**10.** Procédé suivant la revendication 8 ou 9, **caractérisé en ce que** le profil de propriétés viscoélastique des corps géométriques est réglé par la conduite de l'énergie thermique provenant du processus de revêtement, qui comprend l'apport au moins partiel d'énergie de surface ou le prélèvement au moins partiel d'énergie thermique ou une combinaison des deux procédés.

**11.** Procédé suivant les revendications 8 à 10, **caractérisé en ce que** les corps géométriques déposés sur le support auxiliaire présentent au moment du transfert sur le matériau support dans la zone de tête un rapport plasticité/ éiasticité supérieur à 0,3, de préférence de 0,4 à 50, pour une fréquence de 100 rad/s et dans la zone de base un rapport plasticité/élasticité de 0,4 à 50, le rapport plasticité/élasticité dans la zone de tête n'étant pas inférieur à celui de la zone de base.

**12.** Procédé suivant les revendications 8 à 11, **caractérisé en ce que** la masse adhésive thermofusible est sous forme de mousse.

**13.** Procédé suivant les revendications 8 à 12, **caractérisé en ce que** la masse adhésive thermofusible peut être réticulée par un faisceau d'électrons ou par des UV.

**14.** Utilisation suivant une ou plusieurs des revendications précédentes pour des produits médicaux, en particulier des emplâtres, des fixations médicales, des recouvrements de plaies, des systèmes dopés, des bandages orthopédiques ou phlébologiques et des bandes.

**15.** Utilisation suivant la revendication 14, **caractérisée en ce que** le matériau support apprêté de manière auto-adhésive est recouvert après l'application ou est pourvu d'un pansement de plaie ou d'un rembourrage.

**16.** Utilisation suivant la revendication 14, **caractérisée en ce que** le matériau support apprêté de manière auto-adhésive peut être stérilisé, de préférence stérilisé aux rayons γ (gamma).

**17.** Utilisation suivant une ou plusieurs des revendications précédentes pour des fixations réversibles techniques, qui n'autorisent lors de l'enlèvement aucune lésion ou dégradation de divers substrats, comme le papier, les plastiques, le verre, les textiles, le bois, les métaux ou les minéraux.

18. Utilisation suivant une ou plusieurs des revendications précédentes pour des collages techniquement permanents, qui ne peuvent être séparés qu'avec une division partielle du substrat.

Figur 1

Figur 2

Figur 3